# EUROPÄISCHE PATENTSCHRIFT

(11) **EP 1 013 175 B1**
(45) Veröffentlichungstag und Bekanntmachung des Hinweises auf die Patenterteilung: **25.01.2012**
(21) Anmeldenummer: 00100696.4
(22) Anmeldetag: 24.08.1996
(51) Int. Cl.: A23G 3/00, A23L 1/236

(54) **Zuckerfreie dragierte Produkte**
Sugarfree candied product
Produit dragéifié sans sucre

(30) Priorität: 02.09.1995 DE 19532396
(43) Veröffentlichungstag der Anmeldung: 28.06.2000
(62) Teilanmeldung aus: 96930096.1
(73) Patentinhaber: Südzucker Aktiengesellschaft Mannheim/Ochsenfurt, 68165 Mannheim (DE)
(72) Erfinder: Rapp, Knut, M., 67591 Offstein (DE); Willibald-Ettle, Ingrid, 76829 Landau (DE)
(74) Vertreter: Schrell, Andreas

(56) Entgegenhaltungen:
- EP-A- 0 328 849
- EP-A- 0 431 376
- EP-A- 0 625 311
- EP-A- 0 625 578
- WO-A-89/07895
- WO-A-92/13866
- WO-A-95/07622
- DE-A- 3 715 919
- DE-A- 19 523 008
- US-A- 4 317 838
- US-A- 4 792 453
- US-A- 4 961 935
- US-A- 5 525 360
- DATABASE WPI Week 8748 Derwent Publications Ltd., London, GB; AN 87-337070 XP002022953 & JP 62 148496 A (MITSUI SEITO), 2. Juli 1987 (1987-07-02)
- DATABASE WPI Week 8748 Derwent Publications Ltd., London, GB; AN 87-337070 XP002022954 & JP 62 148496 A (MITSUI SEITO), 2. Juli 1987 (1987-07-02)

## Beschreibung

Die vorliegende Erfindung betrifft verbesserte zukkerfreie dragierte Produkte und deren Herstellung.

Dragierte Produkte enthalten eine aus Zucker, Zukkeralkoholen, Schokoladearten und/oder anderen Glasuren hergestellte Decke und einen flüssigen, weichen oder festen Kern. Als Kerne werden zum Beispiel Kaugummi-Einlagen, Früchte, Komprimate oder auch pharmazeutische Produkte verwendet. So beschreibt US 4,792,453 einen zuckerfreien beschichteten Kaugummi, dessen Decke hydrierte Isomaltulose enthält. Dieses Kaugummi wird durch Dragieren mit einem Sirup gewonnen, der hydrierte Isomaltulose enthält. In dem Dragiersirup liegt somit 1-O-α-D-Glucopyranosyl-D-mannit (1,1-GPM) und 6-0-α-D-Glucopyranosyl-D-sorbit (1,6-GPS) gelöst in etwa äquimolaren Mengen vor.

Eine Reihe von Verfahren zur Herstellung dragierter Produkte sind ebenfalls bekannt. Im wesentlichen wird zwischen der Weichdragierung, der Hartdragierung und der Suspensionsdragierung unterschieden. Unter Weichdragierung wird das Aufbringen von in Wasser gelösten Sacchariden auf in Bewegung befindliche Kerne verstanden, wobei nach jedem Auftrag mit Saccharid-Pulver abgestreut wird, um die Feuchtigkeit zu binden. Durch diese Art der Dragierung entsteht eine weiche Dragée-Decke (EP-A1 625 311). Als nachteilig erweist sich neben der komplizierten verfahrenstechnischen Durchführung die Tatsache, daß die Dosierung des zur Dragierung verwendeten Sirups, also des gelösten Saccharids, und die Dosierung des Pulvers aufeinander abgestimmt werden muß.

Unter Hartdragierung wird ebenso wie bei der Weichdragierung das Aufbringen von in Wasser gelösten Sacchariden auf in Bewegung befindliche Kerne verstanden, wobei jedoch kein Saccharid-Pulver aufgetragen wird, sondern unmittelbar die nichtwässrigen Bestandteile angetrocknet werden. Wie bei der Weichdragierung wird eine Vielzahl verschiedener Einzelaufträge (50 bis 120) durchgeführt, zwischen denen mit Warm- oder Kaltluft getrocknet wird, so daß unterschiedlich dicke Dragée-Decken hergestellt werden können. Bekannt sind auch Hartdragierungsverfahren mit zwei unterschiedlichen Saccharidlösungen, die nacheinander aufgetragen werden ("dual composition coating"). So wurden in neuerer Zeit Verfahren beschrieben, in denen zunächst Maltit enthaltende Schichten aufgebracht wurden und anschließend der restliche Dragéeaufbau mit Xylit erfolgte (US 5,376,389). Diese Verfahren verwenden jedoch zwei unterschiedliche Saccharide zur Herstellung der aufzutragenden Lösungen und sind dementsprechend kompliziert durchzuführen. Zudem lösen sich Dragierschichten aus Xylit beim Dragiervorgang insbesondere an Ecken und Kanten der beschichteten Kerne leicht ab.

Sowohl im Hartdragier- als auch im Weichdragierverfahren stellt sich beispielsweise bei der Verwendung von hydrierter Isomaltulose zum Dragieren das Problem der Klebeneigung beim Auftragen der wässrigen Lösungen. Diese Klebeneigung verursacht ein Zusammenkleben des Dragierguts beziehungsweise ein Anhaften an der Dragierkesselwand.

Eine dritte Möglichkeit zur Dragierung besteht in der Verwendung einer Suspension. Die bisher hauptsächlich nur bei zuckerhaltigen Produkten angewandte suspendierte Mischung besteht aus einer flüssigen Phase (die zum Beispiel Zucker, Reisstärke und Glucose gelöst in Wasser enthält) sowie einer festen Phase, die aus feinen kristallinen Zuckerteilen besteht. Charakteristisch für diese Art der Suspensions-Dragierung ist der getrennte Einsatz unterschiedlicher Saccharide.

Die durch die beschriebenen Verfahren erhaltenen Dragierprodukte neigen aufgrund der Zusammensetzung ihrer Decke und ihres Kernes dazu, während der Lagerung ihre Knusprigkeit zu verlieren. Die Ursache dafür liegt vermutlich in der Diffusion von Feuchtigkeit aus dem Kern in die Decke. Dieser Vorgang führt letztendlich zum ebenfalls unerwünschten Austrocknen der Dragéekerne. Umgekehrt weisen die bekannten Produkte in feuchtwarmer Atmosphäre eine unerwünschte Wasseraufnahme auf, deren Resultat klebrige, weiche und damit für den Verzehr unattraktive Produkte sind.

Die Erfindung stellt Verfahren zur Herstellung 1,6-GPS angereicherter Gemische aus 1,6-GPS und 1,1-GPM in einem Verhältnis von größer 57 Gew.-% : kleiner 43 Gew.-% bis 99 Gew.-% 1 Gew.-% (bezogen auf die Trockensubstanz des zur Herstellung eingesetzten Gemisches aus 1,6-GPS und 1,1-GPM, wobei dessen 1,6-GPS/1,1-GPM Gehalt gleich 100% ist) sowie 1,1-GPM angereicherter Gemische aus 1,6-GPS und 1,1-GPM in einem Verhältnis von 1 Gew.-% : 99 Gew.-% bis kleiner 43 Gew.-% : größer 57 Gew.-% (bezogen auf die Trockensubstanz des zur Herstellung eingesetzten Gemisches aus 1,6-GPS und 1,1-GPM, wobei dessen 1,6-GPS/1,1,GPM Gehalt gleich 100% ist) bereit. Die Gemische können je nach Zusammensetzung der für deren Herstellung verwendeten Ausgangssubstanz auch geringe Mengen an Sorbit, Mannit etc. enthalten. Die erfindungsgemäß hergestellten Gemische können in besonders vorteilhafter Weise in Lösung oder als Suspension zur Dragierung von Produkten im Süßmittel- beziehungsweise Arzneimittelbereich verwendet werden. Die Gemische können Zusatzstoff, wesentlicher Bestandteil oder im wesentlichen alleiniger Bestandteil verschiedenster Produkte im Lebensmittel- oder Arzneimittelbereich sein. Es ist besonders vorteilhaft, daß die erfindungsgemäß hergestellten 1,6-GPS und 1,1-GPM angereicherten Gemische aus einer einzigen Grundsubstanz, nämlich hydrierter Isomaltulose, hergestellt werden. Aus dieser kommerziell erhältlichen Grundsubstanz lassen sich also erfindungsgemäß zwei Gemische mit jeweils unterschiedlichen Eigenschaften herstellen. Das 1,6-GPS angereicherte Gemisch zeichnet sich gegenüber hydrierter Isomaltulose und dem 1,1-GPM angereicherten Gemisch durch eine erhöhte Löslichkeit und größere Süßkraft aus. Die größere Süßkraft beruht einerseits darauf, daß 1,6-GPS schneller in Lösung geht und damit ein schnelles Süßempfinden auslöst und andererseits auf der objektiv größeren, der Verbindung 1,6-GPS eigenen Süßkraft. Das 1,1-GPM angereicherte Gemisch weist geringere Löslichkeit als hydrierte Isomaltulose auf. Die gezielte Verwendung dieser beiden Gemische in Produkten im Lebensmittel-, Süßmittel- oder Arzneimittelbereich ermöglicht es, den Produkten eine verbesserte Haltbarkeit und größere Süßkraft zu verleihen sowie deren Herstellungsverfahren zu vereinfachen.

Die Erfindung betrifft insbesondere dragierte Produkte umfassend einen Kern und eine Decke, wobei die Decke wenigstens eine Schicht aus einem 1,6-GPS angereicherten Gemisch aus 1,6-GPS und 1,1-GPM in einem Verhältnis von größer 57 Gew.-% : kleiner 43 Gew.-%, bis 99 Gew.-% : 1 Gew.-% (bezogen auf die Tockensubstanz des zur Herstellung eingesetzten Gemisches aus 1,6-GPS und 1,1-GPM, dessen 1,6-GPS/1,1-GPM Gehalt gleich 100% ist) enthält. Diese Schicht(en) umfassen entweder ausschließlich, gegebenenfalls unter Einschluß von sich aus der Ausgangssubstanz ergebenen Verunreinigungen wie Sorbit oder Mannit, das Gemisch oder dieses Gemisch enthaltende Zusammensetzungen. Die erfindungsgemäßen dragierten Produkte enthalten entweder einen Kern bekannter Zusammensetzung oder einen Kern, der eines oder beide der 1,6-GPS beziehungsweise 1,1-GPM angereicherten Gemische umfaßt, sowie eine Decke aus mindestens einer Schicht aus 1,6-GPS angereichertem Gemisch.

Auch im Arzneimittelbereich spielt die Löslichkeit von Produkten oder, im Falle dragierter Produkte, von deren Decken vielfach eine bedeutende Rolle. Die Löslichkeit der Produkte beziehungsweise Decken beeinflußt unmittelbar die Wirkstoff-Freisetzung und damit auch den Wirkort und die Wirkzeit der applizierten Pharmazeutika. In Fällen, in denen eine raschere Freisetzung der Pharmazeutika erwünscht wird, werden erfindungsgemäß ausschließlich oder vorwiegend 1,6-GPS angereicherte Gemische mit ihrer erhöhten Löslichkeit als Decke oder zur Herstellung des Arzneimittelträgers verwendet.

Die Erfindung betrifft auch ein Verfahren zur Herstellung eines dragierten Produktes, das dadurch gekennzeichnet ist, daß mindestens einmal eine Lösung oder Suspension des 1,6-GPS angereicherten Gemisches auf den Kern aufgetragen wird und nach dem Auftragen jeder Schicht das Lösungsmittel verdampft wird. Das erfindungsgemäße Verfahren sieht vor, daß entweder eine Lösung oder, besonders bevorzugt, eine Suspension des Gemisches mindestens einmal auf einen Kern aufgetragen wird. Besonders bevorzugt ist das mehrmalige Auftragen, so daß die Decke mehrere Schichten umfaßt. Besonders bevorzugt ist ein Verfahren, in dem Schichten beider erfindungsgemäß hergestellter Gemische nacheinander auf den Kern aufgetragen werden. In einer Ausführungsform der Erfindung wird vorgesehen, daß jeweils 25 bis 45 Aufträge der Lösung oder Suspension des 1,1-GPM und 1,6-GPS angereicherten Gemisches durchgeführt werden. Je nach gewünschter Eigenschaft des dragierten Produktes können beispielsweise zuerst die Schichten, die das 1,1-GPM angereicherte Gemisch enthalten, aufgetragen werden, die dann von 1,6-GPS angereicherten Schichten überdeckt werden. Die Erfindung umfaßt jedoch auch das Auftragen zunächst von 1,6-GPS angereicherten Schichten, gefolgt vom Auftragen 1,1-GPM angereicherter Schichten. Nach dem Auftragen jeder Schicht wird das Lösungsmittel verdampft, vorzugsweise mit einem Gasstrom, der einen Taupunkt von -15° bis +10°C besonders bevorzugt 0°C aufweist. Das Aufbringen der Suspension beziehungsweise Lösung wird unter Konstanthaltung der Temperatur und unter Vermeidung von Wasserverlusten durchgeführt. Dies kann beispielsweise in einem DRIACOATER 1200 der Firma Driam, Eriskirch, vollautomatisch erfolgen, wobei die Suspension durch Schlick-Flachstrahldüsen, Durchmesser 1,5 bis 2,0 mm aufgesprüht wird.

In einer bevorzugten Ausführungsform der Erfindung enthält das erfindungsgemäß eingesetzte Gemisch, insbesondere die dieses Gemisch enthaltene Decke der dragierten Produkte, zusätzlich Gummi arabicum in einer Menge von 0,5 Gew.-% bis 10 Gew.-% bezogen auf die Trockensubstanz der Decke. Die Decke umfaßt erfindungsgemäß 10 bis 90 Gew.-%, insbesondere 25 bis 35 Gew.-% der Trockensubstanz des dragierten Produktes. Das erfindungsgemäß eingesetzte Gemisch und insbesondere die Decke damit dragierter Produkte können Farbstoffe, insbesondere Titandioxid enthalten.

In einer weiteren Ausführungsform der Erfindung enthält das Gemisch und daher auch die Decke zusätzlich ein oder mehrere Zuckeraustauschstoffe, insbesondere Xylit, Mannit, Sorbit, Maltit, Lactit oder Erythrit. Erfindungsgemäß ist es auch vorgesehen, daß das Gemisch beziehungsweise die Decke zusätzlich Füllstoffe, insbesondere Polydextrose, Calciumcarbonat oder Inulin enthalten können.

Die vorliegende Erfindung umfaßt auch das Gemisch und dieses enthaltene Decken, die oberflächenaktive Substanzen wie Polysorbate (ethoxylierte Sorbitanester), insbesondere in einer Menge von 0,05 Gew.-% bis 0,5 Gew.-% und/oder Filmbildner wie Methylcellulose-Gelatine, Hydroxypropyl-Cellulose, Ethyl-Cellulose, Hydroxyethyl-Cellulose, Carboxymethyl-Cellulose und Gemische davon enthalten. Zusätzlich können Bindemittel wie Alginate, Pflanzengummis oder Weichmacher vorhanden sein.

In einer weiteren Ausführungsform betrifft die Erfindung das erfindungsgemäß hergestellte Gemisch und die dieses Gemisch enthaltenen Decken dragierter Produkte, die Intensivsüßstoffe, insbesondere Cyclamat, Saccharin, Aspartam, Glycyrrhizin, Dihydrochalcon, Thaumatin, Monellin, Acesulfam, Alitam oder Sucralose enthalten. Insbesondere betrifft die Erfindung ein dragiertes Produkt, dessen Kern ein Kaugummi-Kissen, eine Kaugummi-Kugel, eine Frucht, Nuß, Schokolinse, Hartkaramelle, Weichkaramelle, Gelee, Nonpareille, Liebesperle, pharmazeutisches Produkt oder sonstiges stückiges Lebensmittelprodukt ist.

Der Kern kann dabei bekannter Natur sein oder ein 1,6-GPS beziehungsweise 1,1-GPM angereichertes Gemisch aus 1,6-GPS und 1,1-GPM enthalten. Erfindungsgemäß kann selbstverständlich vorgesehen sein, daß dieser Kern auch die für die Decke vorgesehenen Farbstoffe, Bindemittel, Zuckeraustauschstoffe, Intensivsüßstoffe oberflächenaktive Substanzen oder Füllstoffe enthält. Die Erfindung umfaßt auch ein dragiertes Produkt mit einem Kern in Form eines Komprimates. Das Komprimat kann 1,6-GPS oder 1,1-GPM angereichertes Gemisch enthalten oder ein Komprimat beider Gemische darstellen. Die Auswahl und Menge des für dieses Komprimat verwendeten erfindungsgemäß hergestellten Gemisches beeinflußt dessen Löslichkeit und damit gegebenenfalls auch die von in dem Komprimat enthaltenen Pharmazeutika.

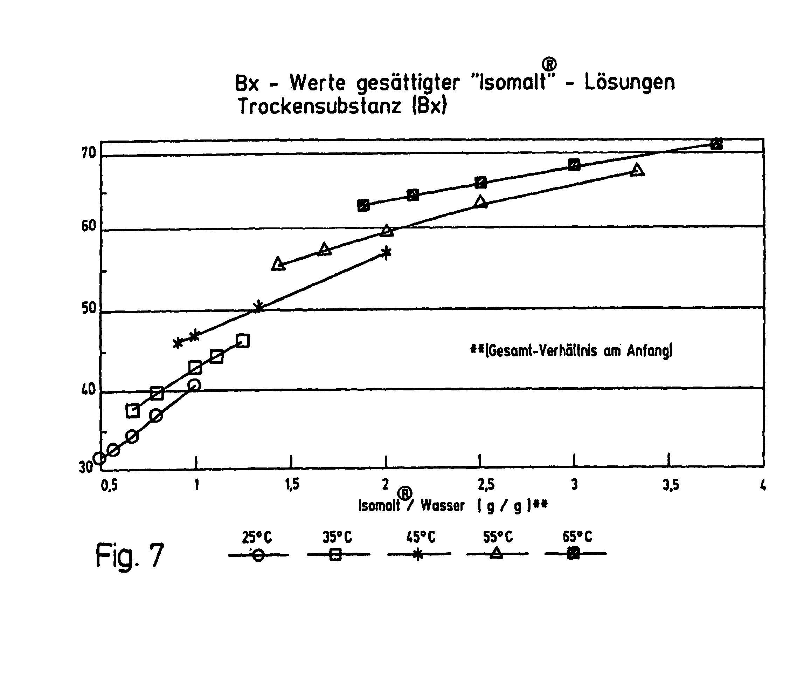

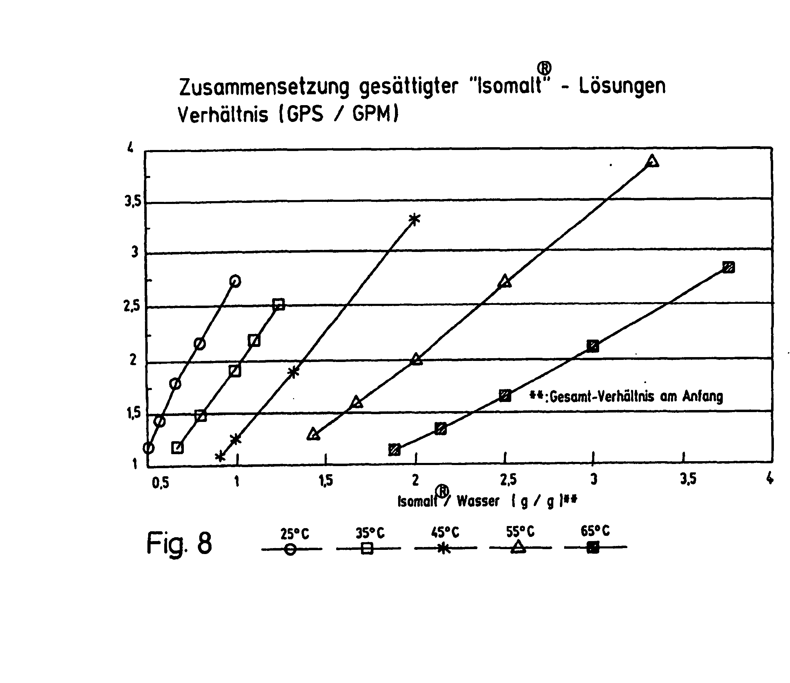

Die Erfindung stellt auch ein Verfahren zur Herstellung eines 1,6-GPS und eines 1,1-GPM angereicherten 1,6-GPS und 1,1-GPM enthaltenen Gemisches aus einem einzigen Ausgangsstoff, nämlich hydrierter Isomaltulose bereit, das dadurch gekennzeichnet ist, daß hydrierte Isomaltulose in Wasser gelöst wird, kristalline hydrierte Isomaltulose in einer Menge zugesetzt wird, daß deren Löslichkeit überschritten wird, die entstandene Suspension gefiltert und das 1,6-GPS angereicherte Filtrat vom 1,1-GPM angereichertem Filterkuchen abgetrennt wird (s. Figur 7 und 8).

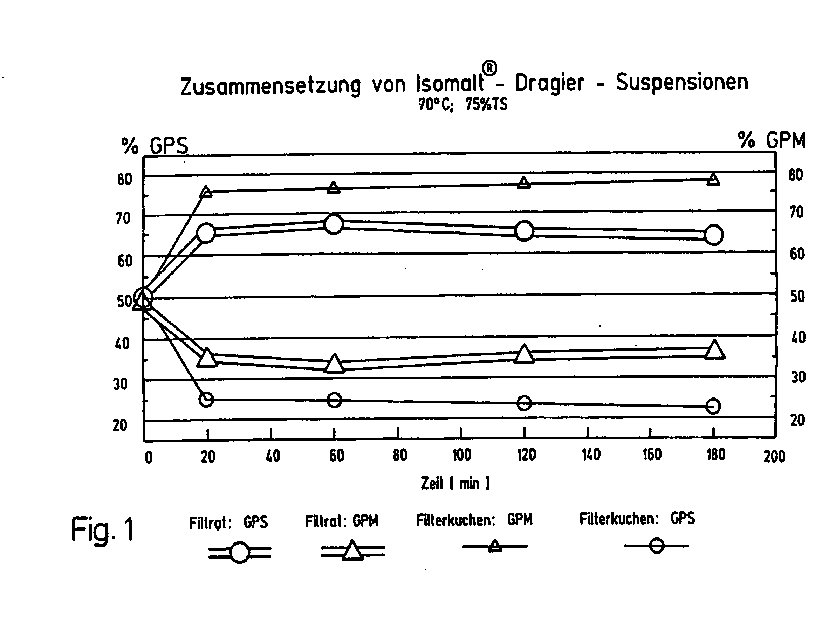

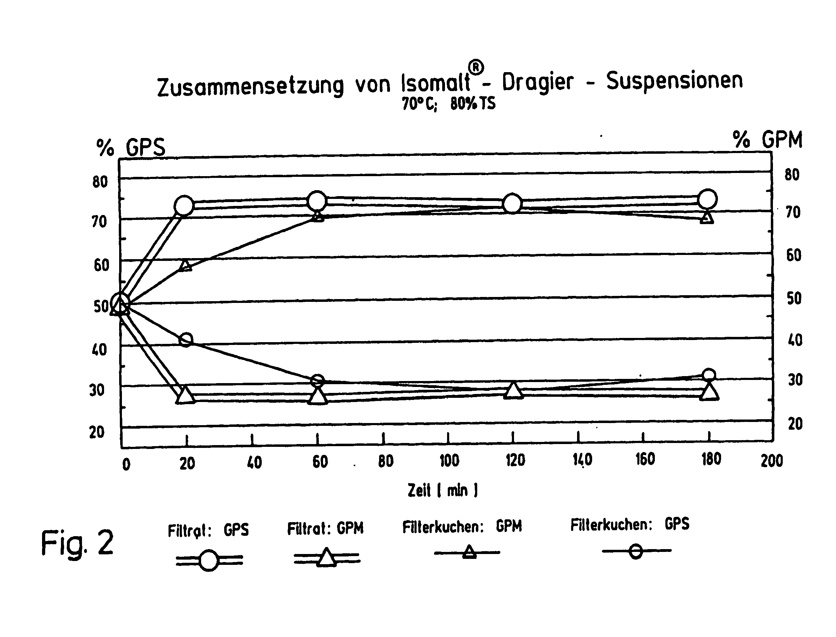

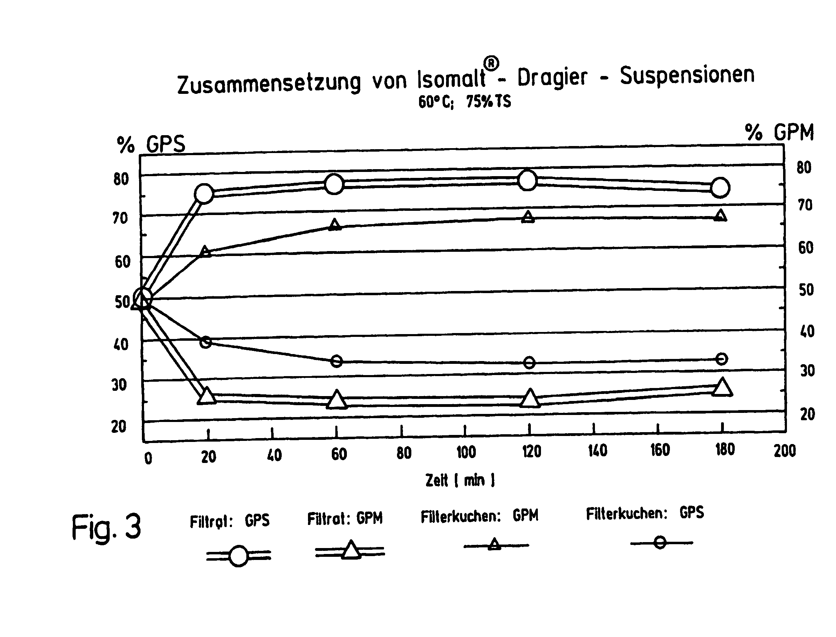

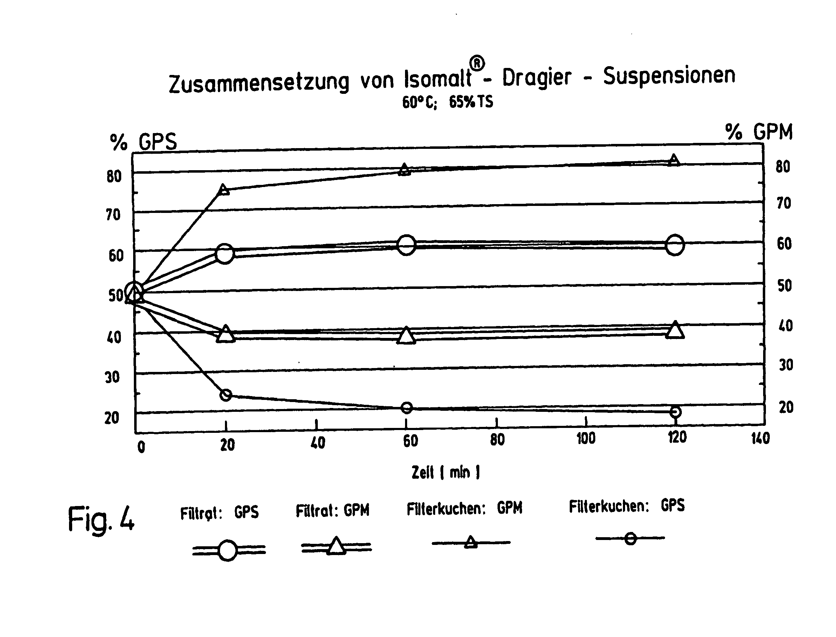

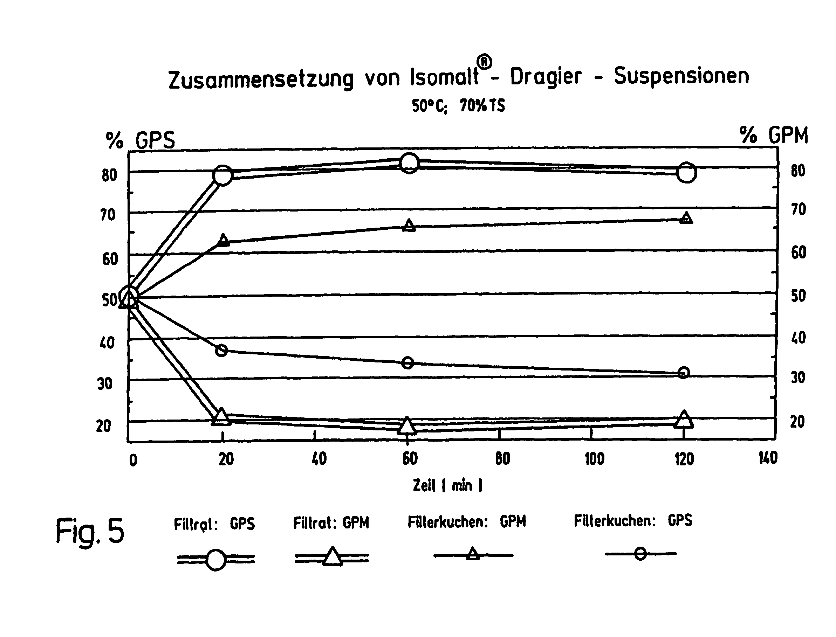

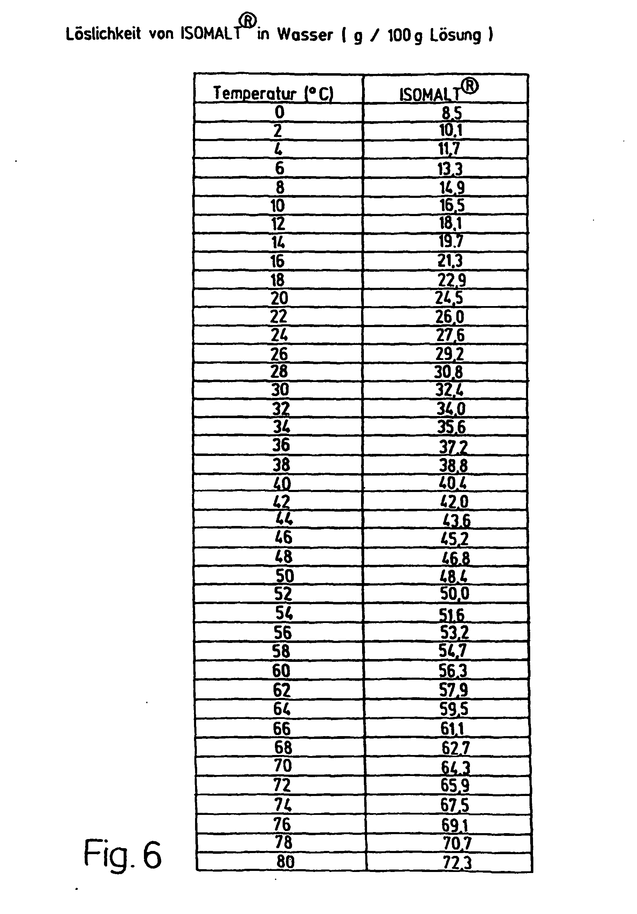

Dieses Verfahren zeichnet sich also dadurch aus, daß zur Herstellung der beiden erfindungsgemäß hergestellten Gemische lediglich ein Ausgangsstoff, nämlich hydrierte Isomaltulose verwendet wird. Hydrierte Isomaltulose ist unter dem Handelsnamen Palatinit^{R} oder ISOMALT^{R} bei der Palatinit GmbH, Mannheim, erhältlich. Sie enthält mehr als 98% 1,6-GPS und 1,1-GPM, wobei Beimengungen von Sorbit oder Mannit möglich sind. In Zusammenhang der vorliegenden Erfindung beziehen sich Anreicherungen von 1,6-GPS und 1,1-GPM immer auf die eingesetzte Menge 1,6-GPS und 1,1-GPM gleich 100%. Erfindungsgemäß wird, insbesondere bei einer Temperatur von 20° bis 95°, eine gesättigte Lösung beispielsweise aus ISOMALT^{R} Typ M hergestellt. Zu dieser Lösung wird in Abhängigkeit von der verwendeten Temperatur feste, pulverförmige hydrierte Isomaltulose, zum Beispiel ISOMALT^{R} Typ PF (Pulver) zugegeben und zwar in einer solchen Menge, daß die in Figur 6 beschriebene Löslichkeit um 1% bis 40% überschritten wird. Die in die wässrige Lösung hydrierter Isomaltulose gegebene kristalline hydrierte Isomaltulose erfährt also Bedingungen, unter denen eine vollständige Lösung der hydrierten Isomaltulose nicht mehr möglich ist. Es bildet sich daher eine Suspension. Der Gesamtfeststoffgehalt dieser Suspension kann dabei bei ca. 50 Gew.-% bis 90 Gew.-% liegen, wobei die Feststoffe teilweise gelöst und teilweise ungelöst vorliegen. Bei der Herstellung der Suspension ist vorzugsweise gut zu rühren. Die Einstellung eines Gleichgewichtes zwischen der Zusammensetzung der flüssigen und der festen Phase der Suspension hängt von dem Gesamtfeststoff-Anteil und der Temperatur ab und ist nach ca. 10 bis 60 min vollzogen. Nach dem Einstellen dieses Gleichgewichts liegt eine flüssige Phase vor, die 1,6-GPS und 1,1-GPM in einem anderen Verhältnis als in hydrierter Isomaltulose enthält. Die suspendierte feste Phase enthält 1,6-GPS und 1,1-GPM ebenfalls in einem anderen Verhältnis als in hydrierter Isomaltulose. Zudem liegt 1,1-GPM in der festen Phase im Gegensatz zum 1,1-GPM in der flüssigen Phase als Dihydrat vor. Die Zusammensetzung der flüssigen und der festen Phase, das heißt die Mengenverhältnisse von 1,6-GPS zu 1,1-GPM sind erfindungsgemäß in weiten Grenzen einstellbar und zwar durch die Temperatur der Suspension und den relativen, ungelösten Feststoffanteil. Die Figuren 1 bis 5 verdeutlichen, daß sich durch Einstellen der Temperatur und des relativen, ungelösten Feststoffanteils die Zusammensetzung der erhaltenen Phasen und damit der erfindungsgemäßen Gemische gezielt steuern läßt. Das Gesamtverhältnis (gelöst und fest) von in den beiden Phasen enthaltendem 1,6-GPS zu 1,1-GPM entspricht natürlich dem der eingesetzten, hydrierten Isomaltulose. Ein geringer ungelöster Feststoff-Anteil in der Suspension ergibt eine hohe 1,1-GPM-Dihydrat-Anreicherung in der festen Phase, ein hoher ungelöster Feststoff-Anteil jedoch eine Zusammensetzung ähnlich der der hydrierten Isomaltulose, wobei jedoch 1,1-GPM-Dihydrat in der festen Phase angereichert ist. In der festen Phase ist also immer 1,1-GPM angereichert, wobei das Verhältnis von 1,6-GPS zu 1,1-GPM von 1 Gew.-% : 99 Gew.-% bis kleiner 43 Gew.-% : größer 57 Gew.-% variieren kann. In der flüssigen Phase ist immer 1,6-GPS angereichert, wobei das Verhältnis von 1,6-GPS zu 1,1-GPM von größer 57 Gew.-% : kleiner 43 Gew.-% bis 99 Gew.-% : 1 Gew.-% variieren kann.

Nachdem sich das Gleichgewicht durch Einstellen der Temperatur und des relativen, ungelösten FeststoffAnteils in der gewünschten Weise eingestellt hat, werden die beiden Phasen erfindungsgemäß voneinander abgetrennt. Die erfindungsgemäß vorgesehene Trennung der beiden Phasen erfolgt durch Abfiltrieren. Man erhält eine 1,1-GPM angereichte feste und eine 1,6-GPS angereicherte flüssige Phase, die jeweils 1,6-GPS und 1,1-GPM in unterschiedlichen Mengenverhältnissen umfassen. Die flüssige Phase kann durch Eindampfen in eine feste Phase überführt werden.

Das erfindungsgemäße Verfahren ermöglicht demgemäß die Bereitstellung 1,6-GPS angereicherter Gemische, bestehend aus 1,6-GPS und 1,1-GPM in einem Verhältnis von größer 57 Gew.-% : kleiner 43 Gew.-% bis 99 Gew.-% : 1 Gew.-%. Die Erfindung stellt außerdem 1,1-GPM angereicherte Gemische bereit, bestehend aus 1,6-GPS und 1,1-GPM in einem Verhältnis von 1 Gew.-% : 99 Gew.-% bis kleiner 43 Gew.-% : größer 57 Gew.-%. Diese Gemische können beispielsweise in Form ihrer Lösung zum Dragieren verwendet werden. Anstelle einer Lösung kann auch eine Suspension eingesetzt werden. Die Verwendung einer Suspension aus 1,6-GPS beziehungsweise 1,1-GPM angereicherten Gemischen zum Dragieren von Produkten weist den Vorteil einer stark verminderten Klebeneigung beim Dragieren auf. Zudem ist die Aufbringung von hohen Trockensubstanzgehalten in relativ kurzer Trockenzeit möglich, da ungelöste Feststoffe zusammen mit gelösten Bestandteilen auf das Dragiergut aufgebracht werden. Die vorteilhafte, verminderte Klebeneigung beruht auf dem Vorhandensein von sehr hohen Anteilen an Kristallisationskeimen, die aus 1,1-GPM-Dihydrat und 1,6-GPS bestehen.

Die gezielte Verwendung der unterschiedlichen Zusammensetzungen und der damit verbundenen unterschiedlichen Eigenschaften der erfindungsgemäß hergestellten Gemische erlaubt nicht nur die Herstellung verbesserter glasierter Produkte oder Komprimate, sondern selbstverständlich auch die Herstellung verbesserter Produkte in allen Bereichen, in denen Zucker oder Zuckerersatzstoffe eine Rolle spielen. So kann beispielsweise das 1,6-GPS angereicherte Gemisch als Weichfüllung in Weichkaramellen Anwendung finden. Das 1,6-GPS angereicherte Gemisch kann in den genannten Produkten auch die bisher verwendeten gut löslichen Zuckeraustauschstoffe wie zum Beispiel Maltit ersetzen. Hartkaramellen können unter Einsatz der 1,1-GPM angereicherten Gemische hergestellt werden. Die die erfindungsgemäß hergestellten Gemische enthaltenden Produkte, insbesondere auch die glasierten bzw. dragierten Produkte, können durch geeignete Wahl der Zusammensetzung der verwendeten Gemische und Schichtenfolge so zusammengesetzt sein, daß die Gesamtzusammensetzung des in dem Produkt enthaltenen 1,6-GPS und 1,1-GPM der Zusammensetzung in handelsüblicher hydrierter Isomaltulose (ISOMALT^{R}, Palatinit^{R}) entspricht.

Die Figuren zeigen:
- Figur 1: stellt die Zusammensetzung der 1,6-GPS und 1,1-GPM angereicherten Phasen dar, die aus einer auf 70°C erwärmten Suspen- sion mit einem Trockensubstanzanteil von 75 Gew.% erhalten werden.
- Figur 2: stellt die Zusammensetzung der 1,6-GPS und 1,1-GPM angereicherten Phasen dar, die aus einer auf 70°C erwärmten Suspen- sion mit einem Trockensubstanzanteil von 80 Gew.% erhalten werden.
- Figur 3: stellt die Zusammensetzung der 1,6-GPS und 1,1-GPM angereicherten Phasen dar, die aus einer auf 60°C erwärmten Suspen- sion mit einem Trockensubstanzanteil von 75 Gew.% erhalten werden.
- Figur 4: stellt die Zusammensetzung der 1,6-GPS und 1,1-GPM angereicherten Phasen dar, die aus einer auf 60°C erwärmten Suspen-

- Figur 5: sion mit einem Trockensubstanzanteil von 65 Gew.% erhalten werden. stellt die Zusammensetzung der 1,6-GPS und 1,1-GPM angereicherten Phasen dar, die aus einer auf 50°C erwärmten Suspen- sion mit einem Trockensubstanzanteil von 70 Gew.% erhalten werden.
- Figur 6: stellt die Löslichkeit von hydrierter Isomaltulose (ISOMALT^{R}) in Wasser dar.
- Figur 7: stellt den Zusammenhang zwischen dem Trockensubstanzgehalt (Bx-Wert) mit hy- drierter Isomaltulose (=ISOMALT^{R}) gesät- tigter Lösung und der Anfangskonzentrati- on von hydrierter Isomaltulose in Wasser bei verschiedenen Temperaturen dar.
- Figur 8: stellt den Zusammenhang zwischen dem Ver- hältnis 1,6-GPS und 1,1-GPM in mit hy- drierter Isomaltulose gesättigter Lösung und der Anfangskonzentration von hy- drierter Isomaltulose in Wasser bei verschiedenen Temperaturen dar.

### Beispiel 1

Herstellung 1,1-GPM und 1,6-GPS angereicherter 1,1-GPM/1,6-GPS Gemische bei 70°C (mit Gummi arabicum-Zusatz)

1920 g ISOMALT^{R} Typ M (hydrierte Isomaltulose) und 67,5 g Gummi arabicum (schnell-löslich) werden bei 80°C in 670,8 g Wasser gelöst und anschließend auf 70°C abgekühlt. Unter Rühren werden dazu 341,7 g ISOMALT^{R} PF (Pulver) zugegeben. Der Wassergehalt von 3,5 Gew.-% in ISOMALT^{R} wurde dabei berücksichtigt.

Im vorliegenden Beispiel wurde nach 20, 60, 120 und 180 min die Trennung der festen von der flüssigen Phase durch Filtration über eine auf 70° temperierte Drucknutsche vorgenommen. Die Zusammensetzungen der erhaltenen Phasen sind in folgender Tabelle I dargestellt:

**Tabelle I**

| Probe | 1 | 2 | 3 | 4 |
|---|---|---|---|---|
| Filtrat in g | 85,85 | 100,89 | 130,83 | 100,9 |
| Filterkuchen (feucht) | 37,76 | 51,55 | 54,76 | 32,34 |
| Filterkuchen (trocken) | 34,30 | 48,56 | 52,10 | 29,53 |
| Filtrat (1,6-GPS:1,1_GPM)% | 75,1:24,9 | 76,7:23,3 | 77:23 | 74,5:25,5 |
| Feststoff (1,1-GPM:1,6-GPS)% | 61:39 | 66,5:33,5 | 67,6:32,4 | 67,2:32,8 |

Die Ergebnisse sind in graphischer Form in Figur 1 dargestellt.

Nach 60 min ist in der flüssigen Phase 1,6-GPS auf ca. 75% angereichert, während in der festen Phase 1,1-GPM (ohne Kristallwasser berechnet) zu über 65% angereichert ist.

Die flüssige Phase kann durch Eindampfen oder Herabsetzen der Temperatur in Suspensionsform beziehungsweise in die feste Phase überführt werden.

Durch mehrmalige Wiederholung dieses Suspendierungs-Trennverfahrens mit den jeweils erhaltenen Phasen wird 1,6-GPS beziehungweise 1,1-GPM in reiner Form erhalten. Durch die Wahl geeigneter Temperaturen und Konzentrationen an hydrierter Isomaltulose sowie gegebenenfalls mehrmaliger Wiederholung des Trennverfahrens ist es erfindungsgemäß auch möglich, 1,1-GPM/1,6-GPS angereicherte Gemische gewünschter Zusammensetzung zu erhalten.

### Beispiel 2

Herstellung 1,1-GPM und 1,6-GPS angereicherter 1,1-GPM/1,6-GPS Gemische bei 35°C

5 kg Isomalt^{R} werden zu 5 kg Wasser (vollentsalzt) gegeben. Die Suspension wird bei 35 °C je nach Korngröße 1 - 20 Stunden gerührt.

Anschließend wird diese Suspension bei 35°C in flüssige Phase und feste Phase in einer beheizten Drucknutsche getrennt.

Die klare Lösung wird im Rotationsverdampfer eingedampft und getrocknet, gegebenenfalls anschließend gemahlen.

Man erhält 1,95 kg weißen Feststoff (Wassergehalt vor Trocknen 24,8%, Verhältnis 1,1-GPM : 1,6-GPS 84% : 16%) und 7,86 kg klare Lösung (42,3°Brix, Verhältnis 1,1-GPM : 1,6-GPS 33,5% : 66,5%).

Die Trennung der beiden Phasen kann auch mittels einer Saugnutsche erfolgen.

Die Beispiele 1 und 2 sowie die Figuren 1 bis 5 verdeutlichen, daß durch gezielten Einsatz der Reaktionsparameter Temperatur und Feststoffkonzentration Gemische gewünschter Zusammensetzung erhalten werden können.

Die Figuren 7 und 8 illustrieren diesen erfindungsgemäßen Vorteil.

Diesen Figuren kann entnommen werden, in welchem Verhältnis hydrierte Isomaltulose (ISOMALT^{R}) mit

Wasser gemischt und bei welcher Temperatur diese Suspension gehalten werden muß, um zum Beispiel eine flüssige Phase mit einem bestimmten 1,6-GPS : 1,1-GPM-Verhätnis zu erhalten.

Mischt man zum Beispiel ISOMALT^{R} mit Wasser im Verhältnis von 2:1, so erhält man bei einer Temperatur von 45°C eine ca. 57°-Brix-Lösung mit einem 1,6-GPS : 1,1-GPM-Verhältnis von 77% : 23%, das heißt, 3,3 : 1.

Dasselbe Gemisch führt aber bei 55°C zu einer ca. 59°Brix-Lösung mit einem 1,6-GPS: 1,1-GPM-Verhältnis von 67% : 33%, das heißt, 2 : 1.

### Vergleichs-Beispiel 3

Herstellung dragierter Produkte im Hartdragierverfahren

### Rezeptur:

| | |
|---|---|
| 1,1-GPM angereichtes Gemisch (85%GPM, 15%GPS) | 30 kg |
| Gummi arabicum | 1,25 kg |
| Titandioxid | 0,5 kg |
| Wasser | 18,3 kg |

### Herstellung der Dragierlösung und Dragierverfahren

Das 1,1-GPM angereicherte Gemisch und Gummi arabicum werden bei ca. 85°C in Wasser gelöst, auf 70°C gekühlt und dann Titandioxid darin suspendiert.

Dieses Gemisch wird unter Rühren bei 70°C gehalten und in einem Hartdragierverfahren auf Kaugummi-Kissen aufgetragen (ca. 50 - 80 Einzelaufträge).

Die Kaugummi-Einlagen (60 kg) werden in einem Diacoater 1200 (Firma Driam, Eriskirch) bewegt und mit Luft (Temperatur 25°C, Taupunkt 0°C) im Gegenströmverfahren nach jedem Auftrag für 2 - 5 Minuten getrocknet.

Der Vorteil des eingesetzten zuckerfreien Rohstoffes (1,1-GPM angereichertes Gemisch) besteht insbesondere darin, daß durch die geringe Löslichkeit des auf den Kernen entstehenden 1,1-GPM-Dihydrats eine Sperrschicht um den Kern ausgebildet wird, die die Diffusion von Wasser und anderen flüchtigen Anteilen (Aromen) aus dem Kern verhindert. Dadurch kommt es nicht zum Austrocknen, wie es bei anderen Dragees häufig beobachtet wird. Auch die sensorisch feststellbare Knusprigkeit bleibt länger erhalten.

### Beispiel 4

Herstellung dragierter Produkte im "Dual-composition"-Verfahren

### Rezeptur I:

| | |
|---|---|
| 1,1-GPM angereichertes Gemisch (85% 1,1-GPM, | 15% |
| 1,6-GPS) | 15 kg |
| Gummi arabicum | 0,6 kg |
| Titandioxid | 0,25 kg |
| Wasser | 9,5 kg |

### Rezeptur II:

| | |
|---|---|
| 1,6-GPS angereichertes Gemisch 77% 1,6-GPS, | 23% |
| 1,1-GPM) | 16,9 kg |
| Gummi arabicum | 0,6 kg |
| Titandioxid | 0,25 kg |
| Wasser | 7,25 kg |

### Verfahren

Rezeptur I wird wie im Vergleichs-Beispiel 3 beschrieben hergestellt und auf die Kaugummi-Einlagen aufgesprüht, wobei die Hälfte der Dragéedecke (Schichten direkt auf dem Kern) in 45 Einzelaufträgen aufgebracht wird.

Rezeptur II wird wie Rezeptur I hergestellt, wobei jedoch die Temperatur der Mischung 60°C beträgt. In 35 Einzelaufträgen wird diese Suspension auf die mit Rezeptur I andragierten Kaugummi-Einlagen bis zum gewünschten Dragée-Endgewicht gebracht.

Die Versuchsparameter entsprechen denen des Vergleichs-Beispiels 3.

Das schwerlösliche 1,1-GPM-Dihydrat aus Rezeptur I (siehe auch Vergleichs-Beispiel 3) bildet eine Sperrschicht gegen Feuchtigkeit aus dem Kern. Die 1,6-GPS angereicherte Außenschicht beeinflußt das Süßempfinden positiv, wie sensorische Untersuchungen (Schwellenwertermittlungen, paarweise Unterschiedsprüfung) ergeben.

### Beispiel 5

Herstellung dragierter Produkte im Suspensionsverfahren

Rezeptur

| | |
|---|---|
| 1,6-GPS angereichertes Gemisch (73% 1,6-GPS, 27% 1,1-GPM) | 43,6 kg |
| Wasser | 29 kg |
| Acesulfam K | 0,05 kg |
| Aspartam | 0,05 kg |
| Titandioxid | 1,0 kg |
| Gummi arabicum | 2,05 kg |
| 1,6-GPS angereichertes Gemisch (Pulver, 77% 1,6-GPS, 23% 1,1-GPM) | 24,25 k |

### Herstellung der Suspension

Unter Rühren wird 1,6-GPS angereichertes Gemisch (43,6 kg) und Gummi arabicum in Wasser aufgelöst und die Lösung auf 75°C erhitzt, bis eine kristallfreie Lösung vorliegt; diese Lösung wird auf ca. 60°C abgekühlt, Aspartam, Acesulfam K, Titandioxid und 1,6-GPS angereichertes Gemisch (Pulver) zugegeben, bis eine homogene Masse vorliegt. Die Temperatur der Suspension wird auf 55°C reguliert und während des Prozesses beibehalten.

Das Dragieren erfolgt analog dem unter Vergleichs-Beispiel 3 beschriebenen Verfahren. Es ergibt sich ein hoher Trockensubstanzauftrag pro Zeiteinheit. Unter anderem durch die erhöhte Löslichkeit von 1,6-GPS wird beim Verzehr ein schnelleres Süßempfinden wahrgenommen.

### Beispiel 6

Sensorische Analyse der Süßkraft von 1,1-GPM- beziehungsweise 1,6-GPS angereicherten 1,1-GPM/1,6-GPS-Mischungen

Für die Analyse der Süßkraft wurde als 1,1-GPM angereichertes Gemisch ein Gemisch mit einem Verhältnis von 1,1-GPM /1,6-GPS von 6,79 : 1 verwendet. Als 1,6-GPS angereichertes Gemisch wurde ein Gemisch mit einem Verhältnis von 1,6-GPS/1,1-GPM von 4,51 : 1 verwendet.

Die Gemische wurden den Probanden in Form wässriger Lösungen verabreicht.

### Schwellenwertermittlunq:

### Konzentrationsreihe 1 (1,1-GPM) der Schwellenprüfung

| **Probenkennzeichnung** | **Konzentration [g/100g]** | **Erkennung des Schwellenwertes [%]** |
|---|---|---|
| 220895/1 | 0 | 0 |
| 220895/2 | 2 | 57,14 |
| 220895/3 | 4 | 14,28 |
| 220895/B | 5 | 28,57 |
| 220895/4 | 6 | 0 |

### Tabelle II

### Konzentrationsreihe 2 (1,6-GPS) der Schwellenprüfung

| **Probenkennzeichnung** | **Konzentration [g/100g]** | **Erkennung des Schwellenwertes [%]** |
|---|---|---|
| 220895/5 | 0 | 0 |
| 220895/6 | 2 | 71,43 |
| 220895/7 | 4 | 14,28 |
| 220895/A | 5 | 14,28 |
| 220895/8 | 6 | 0 |

### Tabelle III

Bei Verwendung eines 1,6-GPS angereicherten Gemisches wird eine geringe Zuckeralkoholkonzentration eher erkannt als bei Verwendung eines 1,1-GPM angereicherten Gemisches.

### Unterschiedsprüfung:

Bei der paarweisen Unterschiedsprüfung (Duo-Test) zeigte sich, daß 62,5% (5 von 8 Probanden) die 1,6 GPS-reiche Mischung in einer 10 %iger Konzentration als süßer empfanden und 37,5% (3 von 8 Probanden) die 1,1-GPM-reiche Mischung.

## Patentansprüche

1. Dragierte Produkte umfassend einen Kern und eine Decke, wobei die Decke wenigstens eine Schicht aus einem 1,6-GPS (6-O-α-D-Glucopyranosyl-D-sorbit) angereicherten Gemisch aus 1,6-GPS und 1,1-GPM (1-O-α-D-Glucopyranosyl-D-mannit) in einem Verhältnis von größer 57 Gew.-% :kleiner 43 Gew.-% bis 99 Gew.-% : 1 Gew.-% (bezogen auf die Trockensubstanz des zur Herstellung eingesetzten Gemisches aus 1,6-GPS und 1,1-GPM, wobei dessen 1,6-GPS/1,1-GPM-Gehalt gleich 100 % ist) enthält und wobei die wenigstens eine Schicht entweder ausschließlich das 1,6-GPS angereicherte Gemisch oder dieses enthaltende Zusammensetzungen umfasst.

2. Dragierte Produkte nach Anspruch 1, **dadurch gekennzeichnet, dass** Farbstoffe in der Decke enthalten sind.

3. Dragierte Produkte nach einem der Ansprüche 1 oder 2, **dadurch gekennzeichnet, dass** der Farbstoff Titandioxid ist.

4. Dragierte Produkte nach einem der Ansprüche 1 bis 3, **dadurch gekennzeichnet, dass** Füllstoffe, insbesondere Polydextrose, Inulin oder Calciumcarbonat in der Decke enthalten sind.

5. Dragierte Produkte nach einem der Ansprüche 1 bis 4, **dadurch gekennzeichnet, dass** oberflächenaktive Substanzen, insbesondere Polysorbate und/oder Filmbildner, insbesondere Carboxymethyl-Cellulose, Methylcellulose-Gelatine, Hydroxypropyl-Cellulose, Hydroxyethyl-Cellulose oder Ethyl-Cellulose in der Decke enthalten sind.

6. Dragierte Produkte nach einem der Ansprüche 1 bis 5, **dadurch gekennzeichnet, dass** der Kern ein Kaugummi-Kissen, eine Kaugummi-Kugel, eine Frucht, Nuss, Schokolinse, Hartkaramelle, Weichkaramelle, Gelee, Nonpareilles, Liebesperle, pharmazeutisches Produkt oder stückiges Lebensmittelprodukt ist.

7. Dragiertes Produkt nach einem der Ansprüche 1 bis 6, **dadurch gekennzeichnet, dass** das Produkt Gelatine, Fett oder Fettersatzstoffe enthält.

8. Dragiertes Produkt nach einem der Ansprüche 1 bis 7, **dadurch gekennzeichnet, dass** dieses einen Intensivsüßstoff, insbesondere Cyclamat, Saccharin, Aspartam, Glycyrrhizin, Neohesperidin, Dihydrochalcon, Thaumatin, Monellin, Acesulfam, Alitam oder Sucralose enthält.

9. Verfahren zur Herstellung (i) eines 1, 6-GPS angereicherten Gemisches aus 1,6-GPS und 1,1-GPM in eimen Verhältnis von größer 57 Gew.-% : kleiner 43 Gew.-% bis 99 Gew.-% : 1 Gew.-% (bezogen auf die Trockensubstanz des zur Herstellung eingesetzten Gemisches aus 1,6-GPS und 1,1-GPM, wobei dessen 1,6-GPS/1,1-GPM-Gehalt gleich 100 % ist) und (ii) eines 1,1-GPM angereicherten Gemisches aus 1,6-GPS und 1,1-GPM in einem Verhältnis von 1 Gew-% : 99 Gew-% bis kleiner 43 Gew.-% : größer 57 Gew.-% (bezogen auf die Trockensubstanz des zur Herstellung eingesetzten Gemisches aus 1,6-GPS und 1,1-GPM, wobei dessen 1,6-GPS/1,1-GPM-Gehalt gleich 100 % ist) aus hydrierter Isomaltulose, **dadurch gekennzeichnet, dass** hydrierte Isomaltulose in Wasser gelöst wird, kristalline hydrierte Isomaltulose in einer Menge zugesetzt wird, dass deren Löslichkeit überschritten wird, die entstandene Suspension gefiltert und das 1,6-GPS angereicherte
Filtrat vom 1,1-GPM angereicherten Filterkuchen abgetrennt wird.

10. Verfahren zur Herstellung eines dragierten Produktes nach einem der Ansprüche 1 bis 6, **dadurch gekennzeichnet, dass** mindestens einmal eine Lösung oder Suspension eines Gemisches, bestehend aus 1,6-GPS und 1,1-GPM in einem Verhältnis von größer 57 Gew.-% : kleiner 43 Gew.-% bis 99 Gew.-% : 1 Ges.-% (bezogen auf die Trockensubstanz des zur Herstellung eingesetzten Gemisches aus 1,6-GPS und 1,1-GPM, wobei dessen 1,6-GPS/1,1-GPM-Gehalt gleich 100 % ist) auf den Kern aufgetragen wird und nach dem Auftragen jeder Schicht das Lösungsmittel verdampft wird.

11. Verfahren nach Anspruch 10, **dadurch gekennzeichnet, dass** die jeweils aufgetragene Schicht nach ihrem Auftragen mit einem Gasstrom getrocknet wird, der einen Taupunkt von -15°C bis +10°C, vorzugsweise 0°C aufweist.

## Claims

1. Coated products comprising a core and a coat, whereby the coat contains at least one layer of a 1,6-GPS (6-O-α-D-glucopyranosyl-D-sorbitol)-enriched mixture of 1,6-GPS and 1,1-GPM (1-O-α-D-glucopyranosyl-D-mannitol) at a ratio of more than 57 % by weight : less than 43 % by weight to 99 % by weight : 1 % by weight (with respect to the dry substance of the mixture of 1,6-GPS and 1,1-GPM that is used in the manufacture, whereby the 1,6-GPS/1,1-GPM content thereof is equal to 100%), and whereby the at least one layer contains either the 1,6-GPS-enriched mixture exclusively or compositions containing said 1,6-GPS-enriched mixture.

2. Coated product according to claim 1, **characterised in that** the coat contains dyes.

3. Coated products according to any one of the claims 1 or 2, **characterised in that** the dye is titanium dioxide.

4. Coated products according to any one of the claims 1 to 3, **characterised in that** the coat contains filling agents, in particular polydextrose inulin or calcium carbonate.

5. Coated products according to any one of the claims 1 to 4, **characterised in that** the coat contains surface-active substances, in particular polysorbates and/or film-forming agents, in particular carboxymethyl-cellulose, methylcellulose-gelatin, hydoxypropyl-cellulose, hydroxyethyl-cellulose or ethyl-cellulose.

6. Coated products according to any one of the claims 1 to 5, **characterised in that** the core is a chewing gum pad, a gumball, a fruit, nut, chocolate bean, hard caramel, soft caramel, jelly, nonpareil, sugar pearls, pharmaceutical product or solid food product.

7. Coated product according to any one of the claims 1 to 6, **characterised in that** the product contains gelatin, fat or fat substitutes.

8. Coated product according to any one of the claims 1 to 7, **characterised in that** the product contains an intensive sweetener, in particular cyclamate, saccharine, aspartame, glycyrrhizine, neohesperidine, dihydrochalcone, thaumatin, monellin, acesulfame, alitame or sucralose.

9. Method for manufacturing (i) a 1,6-GPS-enriched mixture of 1,6-GPS and 1,1-GPM at a ratio of more than 57 % by weight: less than 43 % by weight to 99 % by weight : 1 % by weight (with respect to the dry substance of the mixture of 1,6-GPS and 1,1-GPM that is used in the manufacture, whereby the 1,6-GPS/1,1-GPM content thereof is equal to 100%) and (ii) a 1,1-GPM-enriched mixture of 1,6-GPS and 1,1-GPM at a ratio of to 1 % by weight : 99 % by weight to less than 43 % by weight : more than 57 % by weight (with respect to the dry substance of the mixture of 1,6-GPS and 1,1-GPM that is used in the manufacture, whereby the 1,6-GPS/1,1-GPM content thereof is equal to 100%) from hydrogenated isomaltulose, **characterised in that** hydrogenated isomaltulose is dissolved in water, a quantity of crystalline hydrogenated isomaltulose that exceeds the solubility thereof is added, the resulting suspension is filtered, and the 1,6-GPS-enriched filtrate is separated from the 1,1-GPM-enriched filter cake.

10. Method for manufacturing a coated product according to any one of the claims 1 to 6, **characterised in that** a solution or suspension of a mixture consisting of 1,6-GPS and 1,1-GPM at a ratio of more than 57 % by weight : less than 43 % by weight to 99 % by weight : 1 % by weight (with respect to the dry substance of the mixture of 1,6-GPS and 1,1-GPM that is used in the manufacture, whereby the 1,6-GPS/1,1-GPM content thereof is equal to 100%) is applied to the core at least once and **in that** the solvent is evaporated after applying each layer.

11. Method according to claim 10, **characterised in that** each layer applied is dried in a gas flow having a dew point of -15 °C to +10 °C, preferably 0 °C, after applying it.

## Revendications

1. Produits dragéifiés comprenant un noyau et un enrobage, dans lesquels l'enrobage contient au moins une couche d'un mélange enrichi en 1,6-GPS (6-O-α-D-glucopyranosyl-D-sorbitol) de 1,6-GPS et de 1,1-GPM (1-O-α-D-glucopyranosyl-D-mannitol) dans un rapport de plus de 57 % en poids / moins de 43 % en poids à 99 % en poids / 1 % en poids (par rapport à la substance sèche du mélange utilisé pour la fabrication de 1,6-GPS et de 1,1-GPM, dans lesquels sa teneur en 1,6-GPS/1,1-GPM est égale à 100 %) et dans lesquels l'au moins une couche comprend soit exclusivement le mélange enrichi en 1,6-GPS, soit des compositions contenant celui-ci.

2. Produits dragéifiés selon la revendication 1, **caractérisés en ce que** des colorants sont contenus dans l'enrobage.

3. Produits dragéifiés selon l'une quelconque des revendications 1 ou 2, **caractérisés en ce que** le colorant est le dioxyde de titane.

4. Produits dragéifiés selon l'une quelconque des revendications 1 à 3, **caractérisés en ce que** des charges, notamment du polydextrose, de l'inuline ou du carbonate de calcium sont contenues dans l'enrobage.

5. Produits dragéifiés selon l'une quelconque des revendications 1 à 4, **caractérisés en ce que** des substances tensioactives, notamment des polysorbates et/ou des agents filmogènes, notamment de la carboxyméthylcellulose, de la gélatine de méthylcellulose, de l'hydroxypropylcellulose, de l'hydroxyéthylcellulose ou de l'éthylcellulose sont contenues dans l'enrobage.

6. Produits dragéifiés selon l'une quelconque des revendications 1 à 5, **caractérisés en ce que** le noyau est un coeur de chewing-gum, une boule de chewing-gum, un fruit, une noix, une bille en chocolat, du caramel dur, du caramel mou, de la gelée, des non pareilles, des perles en sucre colorées, un produit pharmaceutique ou un produit alimentaire en morceau.

7. Produit dragéifié selon l'une quelconque des revendications 1 à 6, **caractérisé en ce que** le produit contient de la gélatine, de la graisse ou des substituts de graisse.

8. Produit dragéifié selon l'une quelconque des revendications 1 à 7, **caractérisé en ce que** celui-ci contient un édulcorant intense, notamment du cyclamate, de la saccharine, de l'aspartame, de la glycyrrhizine, de la néohespéridine, de la dihydrochalcone, de la thaumatine, de la monelline, de l'acésulfame, de l'alitame ou du sucralose.

9. Procédé de fabrication (i) d'un mélange enrichi en 1,6-GPS de 1,6-GPS et de 1,1-GPM dans un rapport de plus de 57 % en poids / moins de 43 % en poids à 99 % en poids / 1 % en poids (par rapport à la substance sèche du mélange utilisé pour la fabrication de 1,6-GPS et de 1,1-GPM, dans lequel sa teneur en 1,6-GPS/1,1-GPM est égale à 100 %) et(ii) d'un mélange enrichi en 1,1-GPM de 1,6-GPS et de 1,1-GPM dans un rapport de 1 % en poids / 99 % en poids à moins de 43 % en poids / plus de 57 % en poids (par rapport à la substance sèche du mélange utilisé pour la fabrication de 1,6-GPS et de 1,1-GPM, dans lequel sa teneur en 1,6-GPS/1,1-GPM est égale à 100 %) constitué d'isomaltulose hydrogéné, **caractérisé en ce que** de l'isomaltulose hydrogéné est dissous dans de l'eau, de l'isomaltulose hydrogéné cristallin est ajouté en une quantité, que sa solubilité est dépassée, la suspension résultante est filtrée et le filtrat enrichi en 1,6-GPS est séparé des gâteaux de filtration enrichis en 1,1-GPM.

10. Procédé de fabrication d'un produit dragéifié selon l'une quelconque des revendications 1 à 6, **caractérisé en ce qu'**une solution ou suspension d'un mélange constitué de 1,6-GPS et de 1,1-GPM dans un rapport de plus de 57 % en poids / moins de 43 % en poids à 99 % en poids / 1 % en poids (par rapport à la substance sèche du mélange utilisé pour la fabrication de 1,6-GPS et de 1,1-GPM, dans lequel sa teneur en 1,6-GPS/1,1-GPM est égale à 100 %) est appliquée au moins une fois sur le noyau et le solvant est évaporé après l'application de chaque couche.

11. Procédé selon la revendication 10, **caractérisé en ce que** la couche appliquée à chaque fois est séchée après son application avec un courant gazeux qui présente un point de condensation de -15°C à +10°C, de préférence de 0°C.
